# EUROPEAN PATENT APPLICATION

(11) **EP 2 666 464 A1**
(43) Date of publication of application: **27.11.2013**
(21) Application number: 13169107.3
(22) Date of filing: 24.05.2013
(51) Int. Cl.: A61K 9/20, A61K 31/496

(54) **Stable pharmaceutical composition comprising aripirazole**

(30) Priority: 24.05.2012 IN CH20752012
(71) Applicant: Substipharm Developpement, 75016 Paris (FR)
(72) Inventor: Bothra, CHANDANMAL PUKHRAJ, 560 033 Bangalore (IN); Raghupathi, KANDARAPU, HYDERABAD (IN); Srinivasan, R, 607001 TAMILNADU (IN); Rao, MARAM, SAMBASIVA, 522601 Narasaraopet (IN); Berthier, Leopold, 75016 PARIS (FR); Terrassin, Laurent, 33700 MERIGNAC (FR)
(74) Representative: Regimbeau

(57) **Abstract**

The present invention provides a stable pharmaceutical composition comprising aripiprazole, crospovidone having peroxide content of less than 80 ppm, advantageously less than 50 ppm, and eventually other suitable pharmaceutically acceptable excipient, and its use in the treatment of schizophrenia, bipolar disorder, and clinical depression.

## Description

The present invention relates to stable pharmaceutical composition of atypical antipsychotics. Particularly the present invention relates to stable pharmaceutical composition of aripiprazole. More particularly the present invention relates to stable composition of aripiprazole containing less than 0.5% by weight, compared to the total weight of the composition, ofN-oxide impurity.

Aripiprazole is an atypical antipsychotic used in the treatment of schizophrenia, bipolar disorder, and clinical depression. The atypical antipsychotics (AAP) also known as second generation antipsychotics are a group of antipsychotic tranquilizing drugs used to treat psychiatric conditions. The most common atypical antipsychotics currently available in the market are Aripiprazole, Amisulpride, Asenapine, Clozapine, Olanzepine, Paliperidone, Perospirone, Quetiapine, Remoxipride, Risperidone, Sertindole, Sulpiride, Ziprasidone, Zotepine, Lurasidone Mosapramine, Clotiapine and Blonanserin. Aripiprazole originally marketed as Abilify and is available as tablets, orally disintegrating tablets, oral solutions and intramuscular injections. Chemically aripiprazole is 7- {4-[4-(2,3-dichlorophenyl)piperazin-1-yl]butoxy}-3,4-dihydroquinolin-2(1*H*)-one.

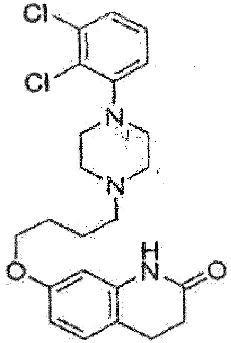

US 2007/0154545 & EP 1 808 165 patents disclose a method of making an aripiprazole formulation comprising a mixture of aripiprazole, at least one diluent, at least one tablet binder, and at least one tablet disintegrant; blending the mixture to obtain a homogeneous mixture; optionally adding at least one tablet lubricant to the homogeneous mixture; and dry compressing the homogeneous mixture into the composition.

US 2007/0154544 & EP 1 808 164 patents disclose a method of making aripiprazole pharmaceutical compositions by wet granulation comprising a mixture of aripiprazole, at least one diluent, at least one tablet binder, and water; blending the mixture to obtain a wet granulate; drying the wet granulate at a temperature of less than 70°C to obtain a dried granulate; and milling the dried granulate to obtain a milled dried granulate, with the proviso that the wet granulate is not milled prior to drying. Further adding at least one tablet lubricant to the milled dried granulate; and compressing the milled dried granulate in a tablet press to obtain a tablet. This patent discloses a method of making aripiprazole tablets by wet granulation method; this method requires more equipment, more time, more space and more labor than the direct compression method.

US 2010/0209495 patent discloses a granulate, where the granule comprises an active pharmaceutical ingredient (API) having a poor water solubility (i.e. less than about 1 mg/mL) which is intimately associated with at least one pharmaceutically acceptable hydrophilic polymer. The granule optionally contains one or more pharmaceutically acceptable excipients, such as disintegrants, wetting agents, diluents, binders, lubricants, glidants, coloring agents and flavoring agents. The invention also relates to a process for preparing the pharmaceutical granulate and pharmaceutical compositions containing the granulate.

EP 2 036 545 patent discloses a method of making tablets by wet granulation method comprising a mixture of aripiprazole (type I and form II), lactose monohydrate, starch, microcrystalline cellulose, hydroxypropyl cellulose, and magnesium stearate. Further the tablets comprise at least one colorant. This patent discloses making of tablets by wet granulation method; this method requires more equipment, more time, more space and more labor than the direct compression method.

AU 2006/224760 & US 2006/0257471 patents disclose a pharmaceutical composition in the form of a tablet by wet granulation method, comprising 1 to 50mg of aripiprazole type II and pharmaceutically acceptable excipients including one or more fillers, one or more binders, one or more disintegrants and a lubricant. These patents disclose the improved stability on storage conditions and does not exhibit a pronounced change in release profile after storage in a variety of conditions than commercial product.

US 2007/0014854 patent discloses a granulate comprises an active pharmaceutical ingredient which has a poor solubility, associated with at least one pharmaceutically acceptable sugar, and at least one pharmaceutically acceptable excipient other than the at least one pharmaceutically acceptable sugar to form a mixture and solution comprises at least one pharmaceutically acceptable sugar and at least one solvent; drying the mixture, comminuting to obtain a pharmaceutical granulate.

US 2010/0004262 patent discloses an orally deliverable pharmaceutical composition for the controlled release of aripiprazole comprising a therapeutically effective amount of aripiprazole and at least one pharmaceutically acceptable excipient, the composition exhibiting an *in-vitro* release profile wherein no more than about 60% of the aripiprazole is dissolved within 3 hours of placement in a standard dissolution test.

US 2010/0297031 patent discloses an orally disintegrating tablet obtainable by direct compression of a dry powdered mixture, said mixture comprising up to 15% by weight of calcium silicate, at least 50% by weight of a diluent, at least a disintegrant; and at least an active ingredient. Said tablets disintegrate quickly in the cavity of the mouth, in particular in less than 15 seconds,

US 2009/0208576 patent discloses a directly compressible composite for an orally disintegrating tablet comprising at least one water-soluble excipient and calcium silicate prepared by co-processing.

EP 1 145 711 patent discloses a flash-melt pharmaceutical dosage form comprising a medicament and a combination of four excipients consisting of a super disintegrant, a dispersing agent, a distributing agent, and a binder. This patent discloses a large amount of dispersing and distributing agent in the formulation which may increase the tablet weight and also increase the formulation cost.

EP 2 359 816 patent discloses a pharmaceutical formulation, characterized by comprising aripiprazole monohydrate and super disintegrant and disintegrant mixture in which the proportion of aripiprazole monohydrate to super disintegrant and disintegrant mixture is in a range of 0.1 to 2.0.
All the above prior art documents disclose various process and compositions for preparation of Aripiprazole dosage forms. None of the above prior art documents discloses information about stability of prepared compositions. The present inventors during development of aripiprazole compositions encountered problem of raising N-oxide impurity during stability and is crossing ICH limits in 3 months of accelerated condition. So there is need to develop stable composition of aripiprazole. The present inventors have developed stable aripiprazole composition comprising crospovidone having peroxide content of less than 80 ppm as disintegrant and one or more pharmaceutical acceptable excipients.

Accordingly, the main embodiment of the present invention provides stable pharmaceutical composition of Aripiprazole, containing less than 0,5% N-oxide impurity by weight, on the basis of the total weight of the composition, where in the composition comprises aripiprazole, crospovidone having peroxide content less than 80 ppm, advantageously less than 50ppm, and one or more suitable pharmaceutically acceptable excipients.

In another embodiment of the present invention, the invention encompasses the use of crospovidone having peroxide content of less than 80 ppm, advantageously less than 50 ppm, in a composition comprising aripiprazole, and eventually other suitable pharmaceutically acceptable excipient, to obtain a stable composition of aripiprazole. Said stable composition of aripiprazole contains less than 0,5% N-oxide impurity by weight, on the basis of the total weight of the composition.

In the description of this invention, the term "N-oxide" represents the N-oxide derivatives of aripiprazole. Said N-oxide derivatives result from the degradation of aripiprazole.

In the description of this invention, the term "stable" means that the quantitative composition does not significantly change over the time, during the entire shelf-life of the composition, namely for at least 3 months, advantageously for at least 6 months, more advantageously for at least 12 months, even more advantageously for at least 36 months, under standard condition, in particular at a temperature ranging for 20°C to 40°C and a relative humidity ranging for 50% to 75%. In particular, the amount of impurities does not increase during the entire shelf-life of the composition. More specifically, N-oxide impurity level is less than 0.5% by weight, based on the total weight on the composition, during the entire shelf life of the composition. In the present invention, the composition is advantageously stable during 6 months at a temperature of 40°C and a relative humidity of 75%.

Furthermore, the phrase "suitable pharmaceutically acceptable excipients", as used herein, means pharmaceutically acceptable excipients having peroxide content less than 80 ppm, advantageously less than 50ppm.

Crospovidone used according to the present invention should have peroxide content of less than 80 ppm, and advantageously less than 50 ppm. Peroxide increases the oxidation of aripiprazole and thereby the N-oxide formation. Thus, a low peroxide content in the composition will reduce oxidation of aripiprazole and thereby reducing N-oxide formation during shelf-life of product. Commonly available crospovidone under the brand names of Kollidon® and Polyplasdone® had peroxide content maximum of 1000 ppm. Thus, said crospovidone will not be able to stop oxidation of aripiprazole during shelf-life and thereby increasing of N-oxide formation during shelf-life and crossing limits mentioned in ICH guidelines Impurities in New Drug products (1CH Q3B(R2) step 4version). Polyplasdone® ultra is a new generation of ultra-pure super disintegrant having significantly lower peroxide content than standard forms of crospovidone.

In the following description, unless otherwise indicated, percentages are expressed by weight on the basis of the total weight of the composition.

The composition according to the present invention comprises between 1% and 20% of aripiprazole, and advantageously between 5% and 15% of aripiprazole.

One or more excipients are advantageously selected from diluents, disintegrants, lubricants, glidants, sweetening agents, flavoring agents and colorants and combinations thereof.

Diluents are inactive ingredients that are added to tablets and capsules in addition to the active drug. A good diluent must be inert, compatible with the other components of the composition, non-hygroscopic, relatively cheap, compatible, and preferably tasteless or pleasant tasting. Suitable diluents according to the present invention are selected from mannitol, micro crystalline cellulose, lactose, sucrose, glucose, sorbitol and dibasic calcium phosphate and combinations thereof. In the present invention, the diluent is present in an amount of about 35% to 85%, advantageously of about 40% to 80%.

Disintegrants are agents added to tablet composition to promote the breakup of the tablet into the small fragments in an aqueous environment thereby increasing the available surface area and promoting a more rapid release of the drug substance. Suitable disintegrants according to the present invention are selected from crospovidone ultra, croscarmellose sodium, croscarmellose calcium, starch, sodium starch glycolate, and alginic acid and combinations thereof. Advantageously, the disintegrant is crospovidone ultra. In the present invention, the disintegrant is present in an amount of about 3% to 15%, advantageously of about 4% to 10%.

Lubricants are agents which prevent ingredients from clumping together and from sticking to the tablet punches or capsule filling machine. Lubricants also ensure that tablet formation and ejection can occur with low friction between the solid and die wall. Suitable lubricants according to the present invention are selected from calcium stearate, magnesium stearate, stearic acid, and talc and combinations thereof. In the present invention, the lubricant is present in an amount of about 0.5% to 5%, advantageously of about 1% to 4%.

Glidants are agents these are used to promote powder flow by reducing inter particle friction and cohesion. Suitable glidants according to the present invention are selected from colloidal silicon dioxide, talc and starch.

Sweetening agents are substances that sweeten medications, food, beverages etc., and suitable sweeteners according to the present invention are selected from aspartame, sucralose, xylitol, sweefil, sweegel, saccharine, sorbitol, maltitol, and combinations thereof. In the present invention, the sweetening agent is present in an amount of about 0.5% to 5%, advantageously of about 1% to 4%.
Flavoring agents are substances added to medicines and foods to improve the quality of taste. Suitable flavoring agents according to the present invention may be selected from vanilla flavor, forest fruit flavor, fruit essences, peppermint oil, spear mint oil, clove oil, orange oil, anise oil and combinations thereof. In the present invention, the flavoring agent is present in an amount of about 0.1 % to 3%, advantageously of about 0.1 % to 2%.

Colorants are agents which gives a special character or distinguishing quality to the pharmaceutical dosage forms. Coloring may be required to increase the aesthetic appearance or identification of a particular composition. Suitable colorants according to the present invention may be selected from indigo carmine lake, iron oxide yellow and iron oxide red and combinations thereof. In the present invention, the colorant is present in an amount of about 0.01 % to 0.5%, advantageously of about 0.01 % to 0.2%.

In an another embodiment, the present invention provides a process for preparing stable compositions of aripiprazole comprising aripiprazole, crospovidone having peroxide content less than 80 ppm, advantageously less than 50 ppm, and one or more suitable pharmaceutically acceptable excipients.

The stable compositions are prepared by techniques known by the skilled person in the art like direct compression or wet granulation or dry granulation. The compositions may be in form of powder and may be filled in capsules or sachets or may be compressed into tablets of immediate release or orally disintegrating or modified release. The composition is advantageously in the form of tablet, and more advantageously in the form of orally disintegrating tablets.

Direct compression technique generally involves blending all ingredients in a blender for suitable time till to achieve blend uniformity and compressing into tablets of suitable size and shape.

Wet granulation technique generally involves utilization of solvents for preparation of granules. This process generally has the steps of mixing active ingredient with diluent, optionally disintegrant, granulating this mixture either by aqueous or non-aqueous granulation, drying the granulate, optionality sieving dried granules and blending dried granules with optionally further diluent, disintegrant, optionally sweetening agent, optionally flavoring agent, optionally colorant and lubricated with lubricant. Suitable solvents used according to the present invention for preparation of wet granulation are selected from water, isopropyl alcohol, methylene chloride and combinations thereof.

Dry granulation is another technique which doesn't use any solvents for preparation of granules. This process generally has the steps of mixing active ingredient with diluent, optionally disintegrant, slugging and de-slugging and blending with optionally sweetening agent, flavoring agent, colorant and finally lubricating with lubricant.

Release modifying agents are used to modify the drug release from the dosage form. Suitable release modifying agents may be selected from hydroxypropyl methylcellulose, hydroxyl propyl cellulose, ethyl cellulose, methyl cellulose, carboxy propyl methyl cellulose, polyethylene oxide, xanthan gum, guar gum, Methacrylates, polyvinyl pyrrolidone, polyvinyl chloride, polypropylene and gelatin.

The stable composition of aripiprazole according to the present invention may be used for the treatment of schizophrenia, bipolar disorder, and clinical depression.

The present invention is further illustrated by the following non limiting examples:

### Examples 1 & 2:

### Compositions of aripiprazole tablets with crospovidone having peroxide content maximum of 400 ppm and above 80 ppm.

### Examples 3&4:

### Composition of aripiprazole tablets with crospovidone having peroxide content of less than 50 ppm.

**Table 1: Compositions of aripiprazole tablets**

| **Examples →** | **Example 1** | **Example 2 (Orally disintegrating)** | **Example 3** | **Example 4 (Orally disintegrating)** |
|---|---|---|---|---|
| **Ingredients ↓** | **mg/Tab** | **mg/Tab** | **mg/Tab** | **mg/Tab** |
| Aripiprazole | 10.00 | 10.00 | 10.00 | 10.00 |
| Mannitol | 78.45 | 76.12 | 78.45 | 76.12 |
| Polyplasdone® XL-10 (having peroxide content maximum of 400ppm and above 80 ppm) | 08.50 | 08.50 | --- | --- |
| Polyplasdone® ultra 10 (having peroxide content of maximum of 50ppm | --- | --- | 08.50 | 08.50 |
| Aspartame | --- | 02.00 | --- | 02.00 |
| Vanilla flavor | --- | 0.33 | --- | 0.33 |
| Iron oxide red | 0.05 | 0.05 | 0.05 | 0.05 |
| Calcium stearate | 3.00 | 3.00 | 3.00 | 3.00 |
| **Total tablet weight** | **100.00** | **100.00** | **100.00** | **100.00** |

### Brief manufacturing process:

### Examples 1 & 3:

i) **Sifting:** mannitol, Polyplasdone® XL-1O/Polyplasdone® ultra 10, and calcium stearate were sifted through #40mesh and iron oxide red was sifted through #1OOmesh separately and collected in polyethylene bag.
ii) **Co-sifting:** Aripiprazole and iron oxide were co-sifted through #20mesh and collected.
iii) **Co-sifting:** Polyplasdone® XL-1O/Polyplasdone® ultra was co-sifted with the mixture of step ii) and blended through #20mesh.
iv) Mannitol was divided into two parts; part I was sifted with the mixture of step iii) and blended through #40mesh.
v) Sifted blend of step iv) was loaded into octagonal blender and part II mannitol was added and the mixture was blended for 10 minutes. Sifted calcium stearate was added to the octagonal blender and blended for 5 minutes.
vi) Blend was compressed into tablets.

### Examples 2 & 4:

This composition was prepared the same as that of example 1 except the aspartame and vanilla flavor were added in step ii) of example 1.

**Packaging:** All the compositions from Examples 1-4 were packaged in Al/Al blisters.

Stability studies: The above packed tablets of Example 1, 2, 3 & 4 were charged for stability studies at 40°C/75% RH (relative humidity) and observed for 6 months. After 6 months, the tablets were analyzed for impurities (Related Substances) content using following validated HPLC method:
Preparation of buffer: Mix 1 ml of Trifluoroacetic acid in to 1000 ml of milli-q-water.
Filter through 0.45 um membrane filter and degas it.

Preparation of mobile Phase A: Mix buffer: methanol in the ratio of 75:25 v/v respectively. Filter through 0.45 µm membrane filter and degas it.

Preparation of mobile Phase B: Mix milli-q-water: methanol: Trifluoroacetic acid in the ratio of 50:50:0.1 v/v/v respectively. Filter through 0.45 µm membrane filter and degas it.

**Chromatographie conditions:**

| HPLC Column | Water Symmetry C18, 150 x 3.9 mm, 5 µm |
|---|---|
| Detector Wavelength | 254 nm |
| Flow rate | 1.5 ml / minute |
| Injection Volume | 25 µl |
| Column temperature | 40°C |
| Run time | About 55 minutes |
| Diluent | Buffer: methanol in the ratio of 75:25 v/v respectively |

**Gradient Program:**

| **Time (mins)** | **Mobile Phase-A** | **Mobile Phase-B** | **Flow Rate (mL/minute)** |
|---|---|---|---|
| 0.01 | 100 | 0 | 1.5 |
| 5.00 | 100 | 0 | 1.5 |
| 10.00 | 10 | 90 | 1.5 |
| 45.00 | 10 | 90 | 1.5 |
| 48.00 | 100 | 0 | 1.5 |
| 55.00 | 100 | 0 | 1.5 |

**The comparative related substances data was given below:**

| **Related substances** | **Acceptance criteria** | **Example 1** | | **Example 2** | | **Example 3** | | **Example 4** | |
|---|---|---|---|---|---|---|---|---|---|
| | | **Initial** | **After 6 months** | **Initial** | **After 6 months** | **Initial** | **After 6 months** | **Initial** | **After 6 months** |
| ARP N-oxide | 0.50% | 0.07% | 0.56% | 0.08% | 0.58% | 0.05% | 0.12% | 0.06% | 0.11% |
| Any unknown impurity | 0.20% | 0.01% | 0.09% | 0.01% | 0.11% | 0.01% | 0.02% | 0.01% | 0.02% |
| Sum of impurities | 1.0% | 0.15% | 0.85% | 0.15% | 0.91% | 0.11% | 0.18% | 0.12% | 0.21% |

From the above data it was clearly shows that in Examples 3 & 4 according to the present invention the content of aripiprazole N-oxide is below 0,5% and thus complies as per the ICH guidelines Impurities in New Drug products.

## Claims

1. Use of crospovidone having peroxide content of less than 80 ppm, advantageously less than 50 ppm, in a composition comprising aripiprazole, and eventually other suitable pharmaceutically acceptable excipient, to obtain a stable composition of aripiprazole.

2. The use according to claim 1, wherein said composition of aripiprazole contains less than 0.5% N-oxide impurity by weight, on the basis of the total weight of the composition.

3. The use according to claim 1 or 2, wherein said composition is stable during at least 3 months, advantageously during at least 6 months, at a temperature of 40°C and a relative humidity of 75%.

4. The use according to any of claim 1 or 3, wherein said excipients are selected from diluents, disintegrants, lubricants, glidants, sweetening agents, flavoring agents and colorants and combinations thereof.

5. The use according to any of claim 1 to 4, wherein said composition is in form of powder.

6. The use according to any of claim 1 to 5, wherein said composition is in form of tablet.

7. The use according to any of claim 1 to 6, wherein said composition is prepared by direct compression or wet granulation or dry granulation.

8. Composition of aripiprazole, wherein the composition comprises aripiprazole, crospovidone having peroxide content of less than 80 ppm, advantageously less than 50 ppm, and eventually other suitable pharmaceutically acceptable excipient.

9. The composition according to claim 8, wherein it contains less than 0.5% N-oxide impurity by weight, on the basis the total weight of the composition.

10. The composition according to claim 8 or 9, wherein it is stable during 6 months at a temperature of 40°C and a relative humidity of 75%.

11. The composition according to any of claim 8 to 10, wherein said excipients are selected from diluents, disintegrants, lubricants, glidants, sweetening agents, flavoring agents and colorants and combinations thereof.

12. The composition according to any of claim 8 to 11, wherein it is in form of powder.

13. The composition according to any of claim 8 to 12, wherein it is formulated as tablet.

14. The composition according to any of claim 8 to 13, wherein it is prepared by direct compression or wet granulation or dry granulation.

15. The composition according to any of claim 8 to 14 for use in the treatment of schizophrenia, bipolar disorder, and clinical depression.
